# EUROPÄISCHE PATENTSCHRIFT

(11) **EP 2 132 165 B1**
(45) Veröffentlichungstag und Bekanntmachung des Hinweises auf die Patenterteilung: **23.04.2014**
(21) Anmeldenummer: 08717224.3
(22) Anmeldetag: 28.02.2008
(51) Int. Cl.: C07C 209/48, C07C 211/48

(54) **VERFAHREN ZUR HERSTELLUNG VON ETHYLENDIAMIN**
METHOD FOR PRODUCING ETHYLENEDIAMINE
PROCÉDÉ DE PRÉPARATION D'ÉTHYLÈNE DIAMINE

(30) Priorität: 01.03.2007 EP 07103290
(43) Veröffentlichungstag der Anmeldung: 16.12.2009
(73) Patentinhaber: BASF SE, 67056 Ludwigshafen (DE)
(72) Erfinder: OFTRING, Alfred, 67098 Bad Dürkheim (DE); DAHMEN, Kirsten, 67251 Freinsheim (DE); HUGO, Randolf, 67246 Dirmstein (DE); HAHN, Thilo, 67292 Kirchheimbolanden (DE); BAUMANN, Katrin, 68529 Mannheim (DE); MELDER, Johann-Peter, 67459 Böhl-Iggelheim (DE)
(86) Internationale Anmeldenummer: PCT/EP2008/052435
(87) Internationale Veröffentlichungsnummer: WO 2008/104592

(56) Entgegenhaltungen:
- US-A- 249 876
- US-A- 3 255 248

## Beschreibung

Die Erfindung betrifft ein Verfahren zur Herstellung von Ethylendiamin durch Hydrierung von Aminoacetonitril an einem Katalysator.

Durch Hydrierung von Aminoacetonitril (AAN) kann Ethylendiamin (EDA) hergestellt werden, welches ein Ausgangsstoff beispielsweise für die Synthese von Komplexbildnern oder Bleichaktivatoren ist, die unter anderem als Waschmittel- oder Reinigungsadditive Verwendung finden.

Es ist generell bekannt, dass Nitrile in Gegenwart von Katalysatoren zu den entsprechenden Aminen hydriert werden können. In Abhängigkeit von den gewählten Reaktionsparametern liefern die bekannten Verfahren die gewünschten Produkte, beispielsweise primäre Amine als Hauptprodukt und sekundäre und tertiäre Amine als Nebenprodukte. Problematisch ist hierbei oft, dass das gewünschte Produkt mit niedriger Selektivität und/oder niedriger Ausbeute anfällt, häufig auch begleitet von einer raschen Inaktivierung des verwendeten Katalysators.

Im Stand der Technik sind zahlreiche Verfahren zur Hydrierung der α-Aminonitrile Aminoacetonitril (AAN) und Iminodiacetonitril (IDAN) oder von β-Aminonitrilen beschrieben. So ist es bekannt, dass die Hydrierung von β-Aminonitrilen in aller Regel problemlos verläuft, während die Hydrierung von α-Aminonitrilen mit dem Auftreten von zahlreichen Nachteilen verbunden ist, wie der Hydrogenolyse der C-CN-Bindung oder der R₂N-C-Bindung. ""Handbook of Heterogeneous Catalytic Hydrogenation for Organic Synthesis, S. 213 bis 215" zeigt die Problematik der Hydrierung von α-Aminonitrilen anhand von α-Alkylaminonitrilen oder cyclischen α-Aminonitrilen im Vergleich zu β-Aminonitrilen auf. Die bekannten Stabilitätsprobleme der α-Aminonitrile sind vermutlich der Hauptgrund dafür, warum bis heute nur die Hydrierung der α-Aminonitrile AAN oder IDAN zu EDA (Ethylendiamin) bzw. DETA (Diethylentriamin) genauer beschrieben wird. Für höhere α-Aminonitrile ist eine entsprechende Hydrierung jedoch nicht bekannt.

Auch die Stabilität von AAN unterscheidet sich noch mal deutlich von der IDAN-Stabilität wie mittels Dynamischer Differenzkalorimetrie gezeigt werden kann. Während der Onset bei IDAN bei 220°C liegt, ist bei AAN schon bei einer Temperatur von 150°C Zersetzung zu beobachten.

Für Verfahren zur Herstellung von Aminen durch Hydrierung von Nitrilen ist zudem bekannt, dass ein gewisser Anteil an Ammoniak die Selektivität der Hydrierung zu primären Aminen begünstigt, und die Bildung von sekundären und tertiären Aminen unterdrückt. Allerdings verursacht die Hydrierung in Gegenwart von Ammoniak zusätzlichen technischen Aufwand, der mit der Abtrennung aus dem Produktstrom, der Aufarbeitung und der eventuellen Rückführung des Ammoniaks verbunden ist. Darüber hinaus können höhere Drücke bei der Hydrierung erforderlich sein, da der Partialdruck des Ammoniaks berücksichtigt werden muss.

In DE-A 3 003 729 wird ein Verfahren zum Hydrieren von aliphatischen Nitrilen, Alkylenoxynitrilen und Alkylenaminonitrilen zu primären Aminen in Anwesenheit eines Lösungsmittelsystems an einem Kobalt- oder Rutheniumkatalysator beschrieben. Das verwendete Lösungsmittelsystem enthält neben Wasser und Ammoniak einen Ether oder Polyether, mit vorzugsweise 4 bis 6 Kohlenstoffatomen, und einem Kohlenstoff zu Sauerstoffverhältnis von 2 : 1 bis 5 : 1, wie Dioxan, Tetrahydrofuran, Methylenglykoldimethylether oder Diethylenglykoldimethylether, wobei cyclische Ether wie Dioxan und Tetrahydrofuran besonders bevorzugt sind. Als Nitrilkomponente sind Dinitrile besonders bevorzugt. In DE-A 3003 729 wird hingegen nicht offenbart, dass auch Verbindungen, die sowohl eine Cyanogruppe als auch eine Aminogruppe aufweisen, wie AAN, in diesem Verfahren verwendet werden können.

EP-A 0 382 508 beschreibt ein Verfahren zur chargenweisen Herstellung von nichtcyclischen, aliphatischen Polyaminen durch Hydrierung von nicht-cyclischen, aliphatischen Polynitrilen in flüssiger Phase an Raney-Kobalt-Katalysatoren, vorzugsweise in Gegenwart von wasserfreiem Ammoniak. Hierbei wird eine Polynitrillösung einer Reaktionszone zugeführt, die den Raney-Kobalt-Katalysator in einer im wesentlichen sauerstofffreien Atmosphäre enthält. Während des gesamten Reaktionszeitraums wird die Polynitrillösung mit einer Rate zugeführt, die nicht größer ist als die maximale Rate, mit der das Polynitril mit dem Wasserstoff in der Reaktionszone reagiert. Es wird weiterhin ein Reaktionsparameter K benannt, welcher zur Bestimmung der volumetrischen Zufuhrrate geeignet ist. Das beschriebene Verfahren beschränkt sich auf die Herstellung von Polyaminen aus Polynitrilen, wie Iminodiacetonitril (IDAN), Nitrilotriacetonitril oder Verbindungen mit mehr als 2 Cyanogruppen. Die Umsetzung von Verbindungen mit einer Cyanogruppe, wie AAN zu EDA, wird jedoch nicht beschrieben.

US-A 3,972,940 betrifft ein Verfahren zur Verhinderung des Aufschäumens der Suspension eines Raney-Nickel- oder Ranay-Kobalt-Katalysators, der bei der katalytischen Hydrierung von Nitrilen zu den entsprechenden Aminen eingesetzt wird. In diesem Verfahren werden sowohl das entsprechende Nitril als auch die Katalysatorsuspension der Reaktionszone kontinuierlich zugeführt. In der Reaktionszone wird das eingesetzte Nitril zum entsprechenden Amin hydriert, nicht umgesetztes Nitril, der Katalysator sowie das Amin, insbesondere Hexamethylendiamin, werden aus der Reaktionszone entfernt. Das Aufschäumen der Katalysatorsuspension wird dadurch unterdrückt, dass der Katalysatorsuspension eine Teilmenge von dem aus der Reaktionszone abgeführten Amin (Produkt) zugeführt wird, bevor die Katalysatorsuspension in die Reaktionszone eingeleitet wird. In US-A 3,972,940 finden sich jedoch keine Angaben, in welcher Rate oder Menge das eingesetzte Nitril der Reaktionszone zugeführt wird. Weiterhin sind weder AAN noch EDA in diesem Dokument explizit aufgeführt.

In US-A 2 519 803 wird ein Verfahren zur Herstellung von Ethylendiamin durch die Hydrierung eines teilweise aufgereinigten wässrigen Reaktionsgemisches beschrieben, welches aus einer Aminierung von Formaldehydcyanhydrin resultiert und als Zwischenprodukt Aminoacetonitril enthält. Das teilaufgereinigte wässrige Reaktionsgemisch wird für maximal 30 Minuten auf ungefähr 5°C abgekühlt bevor es der Hydrierung zugeführt wird. Vorzugsweise findet die Hydrierung in Gegenwart von NH₃ statt.

DE-A 1 154 121 betrifft ein weiteres Verfahren zur Herstellung von Ethylendiamin, wobei die Edukte Blausäure, Formaldehyd, Ammoniak und Wasserstoff in Gegenwart eines Katalysators in einem so genannten Eintopf-Verfahren zur Reaktion gebracht werden. Sowohl der Ammoniak als auch der Wasserstoff werden im molaren Überschuss gegenüber den in äquimolaren Mengen vorliegenden weiteren Edukten Blausäure und Formaldehyd eingesetzt. In diesem Verfahren wird also das in situ gebildete AAN nicht isoliert, sondern direkt mit Wasserstoff weiter umgesetzt. Nachteilig an diesem Verfahren ist, dass das gewünschte Produkte (EDA) relativ unselektiv in kleinen Mengen anfällt.

US-A 3,255,248 beschreibt ein Verfahren zur Hydrierung von organischen Stickstoff-Kohlenstoff-Verbindungen, die vorzugsweise Nitro-, N-Nitroso, Isonitroso, Cyano oder mit Aromaten substituierte Aminogruppen aufweisen, zu den entsprechenden Aminen in flüssiger Phase unter Verwendung eines gesinterten Katalysators aus Kobalt oder Nickel. Hierbei wird das Edukt entweder alleine oder in Gegenwart eines Lösungsmittels, wie beispielsweise Wasser, Tetrahydrofuran, Methanol, Ammoniak oder das gebildete Reaktionsprodukt, gemeinsam mit dem Wasserstoff auf den Katalysator hinuntergerieselt. Sofern am Stickstoffatom ungesättigte Verbindungen, wie Cyanogruppen hydriert werden, wird die Gegenwart von Ammoniak bei der Umsetzung empfohlen. Dies wird in Beispiel 1 dieses Patents verdeutlicht, wo Aminoacetonitril in Form einer wässrigen Lösung mit flüssigem Ammoniak, aber ohne sonstiges Lösungsmittel auf den gesinterten Katalysator gerieselt wird. US-A 3,255,248 enthält jedoch keine Angaben, mit welcher Rate das Edukt zudosiert wird.

EP-A 1 209 146 betrifft ein weiteres Verfahren zur kontinuierlichen Hydrierung von Nitrilen zu primären Aminen, wobei die jeweiligen Nitrile in flüssiger Phase an einem suspendierten, aktivierten Raney-Katalysator auf Basis einer Legierung aus Aluminium eingesetzt werden und die Reaktion in Abwesenheit von Ammoniak und basischen Alkali- oder Erdalkaliverbindungen durchgeführt wird. Als Nitrile können neben vielen anderen auch AAN sowie IDAN zu den entsprechenden Ethylenaminen umgesetzt werden. Gegebenenfalls kann das zu hydrierende Nitril auch in einem organischen Lösungsmittel gelöst vorliegen, wobei vorzugsweise Alkohole, Amine, Amide, insbesondere N-Methylpyrrolidon (NMP) und Dimethylformamid (DMF), sowie Ether oder Ester eingesetzt werden. In EP-A 1 209 146 finden sich jedoch keine Angaben, mit welcher Rate das entsprechende Nitril in das Reaktionsgefäß (Reaktor) zudosiert wird.

Aufgabe der vorliegenden Erfindung ist es demnach, ein einfaches, kostengünstiges und variables Verfahren zur Herstellung von Ethylendiamin durch Hydrierung von Aminoacetonitril bereitzustellen, das Ethylendiamin bei hohem Umsatz und/oder hoher Selektivität liefert.

Die Aufgabe wird von einem Verfahren zur Herstellung von Ethylendiamin durch Hydrierung von Aminoacetonitril an einem Katalysator gelöst, dadurch gekennzeichnet, dass die Hydrierung in einer Lösung durchgeführt wird, welche Aminoacetonitril, Wasser mit in einem Anteil von 0 bis 60 Gew.-% (bezogen auf das Aminonitril-Wassergemisch) und ein Lösungsmittel enthält, wobei das in der Lösung enthaltene Aminoacetonitril mit einer Rate in das Reaktionsgefäß zugeführt wird, die nicht größer ist als die Rate, mit der das Aminoacetonitril mit Wasserstoff bei der Hydrierung reagiert.

Das erfindungsgemäße Verfahren hat den Vorteil, dass Ethylendiamin bei hohem Umsatz und/oder Selektivität hergestellt werden kann. Vorzugsweise wird das eingesetzte Edukt (AAN) vollständig oder nahezu vollständig umgesetzt. Dies ist insbesondere bei großtechnischen Verfahren von Bedeutung, da nicht umgesetztes Edukt in der Regel in den Produktionskreislauf zurückgeführt wird bzw. entsorgt werden muss. Verfahren, bei denen größere Mengen an AAN nicht umgesetzt werden, sind aufgrund der hohen Instabilität des AAN von besonderem Nachteil. Einerseits neigt AAN sich bei höheren Temperaturen zu zersetzen, so dass die Zersetzungsprodukte nicht in den Kreislauf zurückgeführt werden können, andererseits kann diese Zersetzung auch mit explosionsartiger Heftigkeit verlaufen. Da im erfindungsgemäßen Verfahren das AAN vollständig umgesetzt werden kann, müssen hinsichtlich dessen Rückführung in den Produktionszyklus keine Anstrengungen unternommen werden.

Ein weiterer Vorteil des erfindungsgemäßen Verfahrens ist darin zu sehen, dass der eingesetzte Katalysator eine längere Lebensdauer (geringerer Katalysatorverbrauch) aufweist und dass ferner die Katalysatorbelastung höher ist als bei vergleichbaren Verfahren. Aufgrund der höheren Katalysatorbelastung kann ein höherer Raum-Zeit-Ausbeute am Produkt (EDA) erzielt werden.

Das erfindungsgemäße Verfahren geht von dem Edukt Aminoacetonitril (AAN) aus, wobei generell jede Art von AAN eingesetzt werden kann. Vorzugsweise wird destilliertes AAN eingesetzt. Es ist auch denkbar, das erfindungsgemäße Verfahren direkt im Anschluss an die AAN-Synthese durchzuführen, wobei AAN vorzugsweise durch Umsetzung von Formaldehydcyanhydrin (FACH) und Ammoniak nach dem Fachmann bekannten Methoden hergestellt wird. Vorzugsweise wird AAN eingesetzt, das "weitgehend frei von FACH (FACH-frei) ist. Unter weitgehend FACH-frei soll im Rahmen der vorliegenden Erfindung verstanden werden, dass im eingesetzten AAN, bezogen auf die AAN-Menge, maximal 1 Mol% FACH vorhanden ist, vorzugsweise ist AAN vollständig frei von FACH. Ein entsprechend niedriger FACH-Gehalt im AAN kann beispielsweise durch eine hinreichend lange Edukt-Verweilzeit und/oder eine nicht zu tiefe Reaktionstemperatur bei der Umsetzung von NH₃ und FACH erzielt werden. Das AAN wird mit einem Lösungsmittel und eventuell mit Wasser vermischt und die dabei erhaltene Lösung wird im erfindungsgemäßen Verfahren eingesetzt. Die Verwendung eines Lösungsmittels erweist sich als vorteilhaft, da eine Stabilisierung von AAN erreicht und die Zuführung mit einer gewünschten Rate in die Vorrichtung zur Durchführung des Verfahrens vereinfacht werden kann. Außerdem lässt sich dadurch ein Spüleffekt an dem eingesetzten Katalysator erzielen, wodurch dessen Standzeit erhöht (längere Katalysatorlebensdauer) sowie die Katalysatorbelastung verbessert wird.

Ein geeignetes Lösungsmittel, welches ein oder mehrere Komponenten umfassen kann, sollte folgende Eigenschaften aufweisen:
(a) das Lösungsmittel sollte stabilisierend auf das AAN wirken, insbesondere eine Zersetzung des AAN bei den herrschenden Temperaturen unterdrücken;
(b) das Lösungsmittel sollte eine gute Wasserstofflöslichkeit zeigen;
(c) das Lösungsmittel sollte bei den Reaktionsbedingungen inert und einphasig sein;
(d) im Hinblick auf eine bevorzugt destillative Abtrennung des Produktes im Anschluss an die Hydrierung aus dem Produktstrom, sollte das Lösungsmittel keine Azeotrope mit dem bzw. den Produkten bilden;
(e) das Lösungsmittel sollte eine entsprechend niedrige Siedetemperatur aufweisen.

Mögliche Lösungsmittel sind organische Lösungsmittel, beispielsweise Amide, wie N-Methylpyrrolidon (NMP) und Dimethylformamid (DMF), aromatische und aliphatische Kohlenwasserstoffe, wie Benzol und Xylol, Alkohole wie Methanol, Ethanol, Propanol, Isopropanol, Butanol, Isobutanol, sekundäres Butanol und tertiäres Butanol, Amine, wie Ethylenamine, Alkylamine, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF). Bevorzugt werden im erfindungsgemäßen Verfahren Ether verwendet, mehr bevorzugt cyclische Ether und besonders bevorzugt Tetrahydrofuran. In einer weiteren bevorzugten Ausführungsform werden Alkohole, insbesondere Methanol, als organisches Lösungsmittel verwendet.

Das Lösungsmittel wird im Gewichtsverhältnis zu dem eingesetzten Aminonitrilgemisch von 0,1 : 1 bis 15 : 1 eingesetzt. Die Konzentration des Aminonitrilgemisches in der Lösung, in der die Hydrierung durchgeführt wird, sollte so gewählt werden, dass eine geeignete Zuführrate bzw. Verweilzeit eingestellt werden kann. Es ist bevorzugt, Aminoacetonitril zu 10 bis 90 Gew.% mit dem Lösungsmittel zu vermischen. Bezogen auf die besonders bevorzugten Lösungsmittel Methanol bzw. Tetrahydrofuran ist es beispielsweise vorteilhaft, das Aminonitrilgemisch zu 20 bis 50 Gew.-% bezogen auf das Lösungsmittel einzusetzen.

Die für die Herstellung von Ethylenaminen durch Hydrierung des Aminonitrilgemisches eingesetzte Lösung kann neben dem Aminonitrilgemisch und gegebenenfalls dem Lösungsmittel auch einen Anteil an Wasser enthalten. Sofern Wasser vorhanden ist, liegt der gewichtsmäßige Anteil an Wasser in der Lösung in einem Bereich von 0 bis 70 %, vorzugsweise bei 10 bis 50 %. Die Mengenangaben des Wassers beziehen sich dabei auf das Aminonitril-Wasser-Gemisch.

Gegebenenfalls können in der Lösung, in der die Hydrierung durchgeführt wird, zusätzliche Additive enthalten sein. Als Additive kommen prinzipiell Hydroxide wie Alkalimetallhydroxide, Alkoholate, Amide oder Ammoniak in Frage. Weiterhin können auch saure Additive, wie zum Beispiel Silikate zusätzlich in der Lösung enthalten sein. Diese Substanzen können als Reinstoff oder gelöst in einem Lösungsmittel zugesetzt werden. Vorzugsweise wird das erfindungsgemäße Verfahren ohne den Zusatz von Additiven durchgeführt.

In einer bevorzugten Ausführungsform des Verfahrens wird der Lösung, in der die Hydrierung durchgeführt wird, kein Ammoniak zugesetzt. Sofern noch Ammoniak in den Edukten bzw. in der gegebenenfalls eingesetzten wässrigen Lösung gelöst ist bzw. als Nebenprodukt bei der Hydrierung freigesetzt werden, ist dies nicht störend. Gegebenenfalls vorhandener Ammoniak kann nach dem Fachmann bekannten Methoden, beispielsweise destillativ, entfernt werden.

Als Katalysatoren zur Hydrierung der Nitril-Funktion zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten. In einer bevorzugten Ausführungsform werden im erfindungsgemäßen Verfahren Raney-Katalysatoren eingesetzt, bevorzugt Raney-Kobalt- oder Raney-Nickel-Katalysatoren und besonders bevorzugt mit mindestens einem der Elemente Cr, Ni oder Fe dotierte Raney-Kobalt- oder mit einem der Elemente Mo, Cr oder Fe dotierte Raney-Nickel-Katalysatoren.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff-enthaltendem Gasstrom bei erhöhter Temperatur aktiviert. Wenn die Katalysatoren außerhalb des Reaktors reduziert werden, kann danach eine Passivierung durch einen Sauerstoff-enthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können.

Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂,1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als Metallschwamm' aus einer binären Legierung (Nickel, Eisen, Kobalt mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach z. B. mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

Bevorzugt wird erfindungsgemäß ein Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni, Cr enthält.

Solche Katalysatoren enthalten typischerweise neben Kobalt noch 1 - 30 Gew.-% Al, besonders 2 - 12 Gew.-% Al, ganz besonders 3 - 6 Gew.-% Al, 0 - 10 Gew.-% Cr, besonders 0,1 - 7 Gew.-% Cr, ganz besonders 0,5 - 5 Gew.-% Cr, insbesondere 1,5 - 3,5 Gew.-% Cr, 0 - 10 Gew.-% Fe, besonders 0,1 - 3 Gew.-% Fe, ganz besonders 0,2 - 1 Gew.-% Fe, und/oder 0 - 10 Gew.-% Ni, besonders 0,1 - 7 Gew.-% Ni, ganz besonders 0,5 - 5 Gew.-% Ni, insbesondere 1 - 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:
Al: 2-6 Gew.%, Co: ≥ 86 Gew.-%, Fe: 0-1 Gew.%, Ni: 1-4 Gew.-%, Cr: 1,5 - 3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch
1 - 30 Gew.-% Al, besonders 2 - 20 Gew.-% Al, ganz besonders 5 - 14 Gew.-% Al,
0 - 10 Gew.-% Cr, besonders 0,1 - 7 Gew.-% Cr, ganz besonders 1 - 4 Gew.-% Cr, und/oder
0 - 10 Gew.-% Fe, besonders 0,1 - 7 Gew.-% Fe, ganz besonders 1 - 4 Gew.% Fe,
wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden.
Dieser Katalysator weist folgende Zusammensetzung auf
Al: ≤14 Gew.-%, Ni: ≥ 80 Gew.-%, Fe: 1-4 Gew.-%, Cr: 1-4 Gew.-%.
Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Die Temperaturen, bei denen das erfindungsgemäße Verfahren durchgeführt wird, liegen in einem Bereich von 40 bis 150°C, bevorzugt von 40 bis 120°C.

Der bei der Hydrierung herrschende Druck liegt im Allgemeinen bei 5 bis 300 bar, bevorzugt bei 30 bis 250 bar, besonders bevorzugt bei 50 bis 160 bar.

Es konnte ein Zusammenhang zwischen der Zuführrate des Aminoacetonitrils, der Ausbeute an Ethylendiamin und dem Anteil an Ethylendiamin bzw. den Anteilen sekundärer und tertiärer Amine im Produkt gefunden werden. Im erfindungsgemäßen Verfahren wird die Lösung, in der Aminoacetonitril enthalten ist, mit einer Rate in das Reaktionsgefäß zugeführt, die nicht größer als die Rate ist mit der das Aminoacetonitril mit Wasserstoff bei der Hydrierung reagiert. Bevorzugt ist eine Zuführrate des Aminoacetonitrils, die im Wesentlichen der Rate der Hydrierung von Aminoacetonitril mit Wasserstoff angepasst ist. Es ist bevorzugt, dass nur so viel Aminoacetonitril in das Reaktionsgefäß zugeführt wird, wie durch die Reaktion mit Wasserstoff abreagiert. Somit ergibt sich eine Zuführrate der Aminoacetonitril enthaltenen Lösung, welche in Zusammenhang zu der Konzentration des Aminoacetonitrils in der eingesetzten Lösung und zu den Parametern der Reaktionskinetik steht. Diese sind unter anderem die herrschende Temperatur, der herrschende Druck und die Wasserstoffverfügbarkeit, welche auch durch Mischeffekte bei der Reaktion beeinflusst wird. Es ist bevorzugt, das die Konzentration des Aminoacetonitrils im Reaktionsaustrag im Wesentlichen kleiner als 1 Gew.-%, besonders bevorzugt < 1000 ppm ist.

Durch die vorgenannte Einstellung der Zuführrate des Aminoacetonitrils wird eine möglichst schnelle Reaktionskinetik, um die Bildung von Nebenprodukten, beispielsweise sekundären und tertiären Aminen, zu reduzieren, aber auch um die Zersetzung des eingesetzten Aminoacetonitrils und damit der Katalysatorverbrauch zu minimieren.

Auch wenn die Katalysatoraktivität durch die Zugabe von z.B. FACH partiell deaktiviert wird, kann durch Anpassung der Hydrierbedingungen, wie z.B. Erhöhung der Temperatur, die gewünschte Selektivität erzielt werden.

Im erfindungsgemäßen Verfahren kann das Lösungsmittel zunächst vollständig mit AAN vermischt werden. Die Lösung, die gegebenenfalls auch Wasser sowie weitere Additive enthält, wird anschließend in das den Katalysator enthaltende Reaktionsgefäß mit der erforderlichen Rate zugeführt. Gegebenenfalls kann, beispielsweise bei Semibatch-Verfahren, ein Teil des Lösungsmittels zusammen mit dem Katalysator im Reaktionsgefäß vorgelegt werden, worauf die Lösung mit der erforderlichen Rate zudosiert wird. Bei kontinuierlichen Verfahren kann eine Teilmenge des Lösungsmittels auch separat von der Lösung, die AAN, das Lösungsmittel und gegebenenfalls Wasser enthält, in das Reaktionsgefäß zugegeben werden. Da AAN bei Raumtemperatur flüssig ist, kann dieses auch komplett separat zur Hydrierung zudosiert werden. Alternativ ist auch die Zudosierung von AAN als wässrige Lösung mit einer separaten Zudosierung des organischen Lösemittels denkbar.

Das erfindungsgemäße Verfahren zur Herstellung von Ethylendiamin durch Hydrierung von Aminoacetonitril kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des Aminonitrilgemisches und des Katalysators mit dem gasförmigen Wasserstoff unter Druck möglich ist.
Die Hydrierung in Suspensionsfahrweise kann in einem Rührreaktor, Schlaufenreaktor oder sonstigem rückvermischten Reaktor, Strahlschlaufenreaktor, Strahldüsenreaktor, Blasensäulenreaktor, bzw. in einer Kaskade derartiger gleicher oder verschiedener Reaktoren durchgeführt werden. Für die Hydrierung an einem Festbettkatalysator sind Rohreaktoren aber auch Rohrbündelreaktoren denkbar.

Im Fall eines Festbettkatalysator wird in Sumpf- oder Rieselfahrweise mit dem Aminonitrilgemisch beaufschlagt. Bevorzugt wird allerdings die Suspensionsfahrweise in semikontinuierlicher und bevorzugt in kontinuierlicher Fahrweise eingesetzt.

Die Hydrierung der Nitrilgruppen findet unter Freisetzung von Wärme statt, die in der Regel abgeführt werden muss. Die Wärmeabfuhr kann durch eingebaute Wärmeübertragerflächen, Kühlmantel oder außenliegende Wärmeübertrager in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeübertragers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich.

Im Anschluss an die Hydrierung kann das erhaltene Produkt (EDA) gegebenenfalls weiter aufgereinigt werden, beispielsweise indem das verwendete Lösungsmittel, gegebenenfalls Wasser und/oder der Katalysator nach dem Fachmann bekannten Methoden abgetrennt werden. Als Nebenprodukte anfallende Verbindungen wie beispielsweise Diethylentriamin. (DETA) oder sonstige Verunreinigungen können ebenfalls mit dem Fachmann bekannten Methoden abgetrennt werden.

In einer bevorzugten Ausführungsform wird das erfindungsgemäße Verfahren unter Verwendung von Tetrahydrofuran als Lösungsmittel durchgeführt. Die Temperatur bei der Hydrierung beträgt vorzugsweise 75 bis 125°C, der Druck vorzugsweise 50 bis 160 bar. Vorzugsweise wird die Hydrierung in Abwesenheit von Ammoniak durchgeführt.

Mit dem erfindungsgemäßen Verfahren wird eine hohe Katalysatorbelastung erzielt, die ein Maß für die Aktivität des verwendeten Katalysators ist. Bevorzugt beträgt die Katalysatorbelastung 0,3 bis 20 mol Nitril (entspricht ∼0,2g bis 12g AAN/g Kat), vorzugsweise 1 bis 10 mol Nitril (∼0,6g-6g) pro Gramm Katalysator pro Stunde. Je höher die Katalysatorbelastung ist, umso höher kann auch die Raum-Zeit-Ausbeute an Ethylenaminen sein.

### Beispiele

Die nachfolgenden Beispiele verdeutlichen das erfindungsgemäße Verfahren. Die Anteile sind in Gew.-% angegeben, sofern nicht anders aufgeführt. Ein mitgeführter interner Standard, Diethylenglykoldimethylether (DEGDME), erlaubt eine Quantifizierung des Produktes durch Bestimmung der eventuell gebildeten flüchtigen Zersetzungsbestandteile. Die Quantifizierung erfolgt mittels Gaschromatographie (GC), wobei den jeweils entnommenen Proben zur Homogenisierung Methanol zugegeben wird.

### Beispiel 1 (Semibatch)

In einem 270ml-Autoklaven werden 3,25g eines Cr-dotierten Raney-Kobalt-Katalysators sowie 15ml THF vorgelegt. Der Autoklav wird auf 80°C erwärmt und Wasserstoff bis zu einem Gesamtdruck von 200 bar aufgepresst. Innerhalb von 20 Minuten wird eine Mischung aus 13,8g (0,25mol) destilliertem AAN [12,7g/g(kat)/h], 13,8g eines internen Standards sowie 4,2g Wasser in 106g THF zudosiert. Die Reaktionsmischung wird weitere 90 Minuten unter Reaktionsbedingungen gerührt. Zu unterschiedlichen Zeiträumen werden Proben genommen, die mittels Methanol homogenisiert werden. Sofort nach vollständiger Zugabe der AAN-Lösung kann kein AAN mehr nachgewiesen werden. Die Selektivität liegt nach 90minütiger Nachhydrierzeit bei 96 % EDA sowie 2 % DETA.

### Vergleichsbeispiel 2 (Batch)

In einem 270ml-Autoklaven werden 3,25g eines Cr-dotierten Raney-Kobalt-Katalysators sowie 13.8g (0,25mol) destilliertem AAN, 13,8g eines internen Standards sowie 4,2g Wasser und 121ml THF vorgelegt. Der Autoklav wird auf 80°C erwärmt und Wasserstoff bis zu einem Gesamtdruck von 200 bar aufgepresst. Die Reaktionsmischung wird 60 Minuten unter Reaktionsbedingungen gerührt. Der Reaktionsmischung wird mittels Methanol homogenisiert. Es kann kein AAN mehr nachgewiesen werden. Die Selektivität liegt nach 60minütiger Hydrierzeit bei 74% EDA sowie 12% DETA.

Bei einer Batchhydrierung liegt anfänglich eine hohe AAN-Konzentration vor. Demzufolge wird mehr AAN "zudosiert" als hydriert, weshalb die Raktion mit einer geringeren Selektivität abläuft.

### Beispiel 3 (Semibatch)

In einem 270ml-Autoklaven werden 3,25g eines Cr-dotierten Raney-Kobalt-Katalysators sowie 15ml THF vorgelegt. Der Autoklav wird auf 80°C erwärmt und Wasserstoff bis zu einem Gesamtdruck von 50 bar aufgepresst. Innerhalb von 20 Minuten wird eine Mischung aus 13,8g (0,25mol) destilliertem AAN (12,7g/g(Kat)/h, 1/3tel des Druckes im Vergleich zu Beispiel 1), 13,8g eines internen Standards sowie 4,2g Wasser in 106g THF zudosiert. Die Reaktionsmischung wird weitere 90 Minuten unter Reaktionsbedingungen gerührt. Zu unterschiedlichen Zeiträumen werden Proben genommen, die mittels Methanol homogenisiert werden. Nach vollständiger Zugabe kann kein AAN mehr nachgewiesen werden. Die Selektivität liegt nach 90minütiger Nachhydrierzeit bei 90% EDA sowie 4% DETA.

### Vergleichsbeispiel 4 (Semibatch)

In einem 270ml-Autoklaven werden 3,25g eines Cr-dotierten Raney-Kobalt-Katalysators sowie 15ml THF vorgelegt. Der Autoklav wird auf 60°C erwärmt und Wasserstoff bis zu einem Gesamtdruck von 50bar aufgepresst. Innerhalb von 20 Minuten wird eine Mischung aus 13,8g (0,25mol) destilliertem AAN ((12,7g/g(Kat)/h, 20°C geringerer Temperatur im Vergleich zu Beispiel 2), 13,8g eines internen Standards sowie 4,2g Wasser in 106g THF zudosiert. Die Reaktionsmischung wird weitere 90 Minuten unter Reaktionsbedingungen gerührt. Zu unterschiedlichen Zeiträumen werden Proben genommen, die mittels Methanol homogenisiert werden. Nach vollständiger Zugabe kann noch 0,8% AAN nachgewiesen werden. Die Selektivität liegt nach 90minütiger Nachhydrierzeit bei 82% EDA sowie 0,4% DETA.

Unter den gewählten Bedingungen kann am Ende der Zudosierung noch AAN nachgewiesen werden. Somit wird schneller zudosiert als hydriert, was eine geringere Selektivität zur Folge hat.

### Vergleichsbeispiel 5 (partielle Deaktivierunct durch FACH)

In einem 270ml-Autoklaven werden 3,25g eines Cr-dotierten Raney-Kobalt-Katalysators sowie 15ml THF vorgelegt. Der Autoklav wird auf 80°C erwärmt und Wasserstoff bis zu einem Gesamtdruck von 50 bar aufgepresst. Innerhalb von 20 Minuten wird eine Mischung aus 13,8g (0,25mol) destilliertem AAN [12,7g/g(kat)/h]" 13,8g eines internen Standards sowie 4,2g Wasser, sowie 69mg an Formaldehydcyanhydrin in 106g THF zudosiert. Die Reaktionsmischung wird weitere 90 Minuten unter Reaktionsbedingungen gerührt. Zu unterschiedlichen Zeiträumen werden Proben genommen, die mittels Methanol homogenisiert werden. Nach vollständiger Zugabe kann noch 0,7% AAN. mehr nachgewiesen werden. Die Selektivität liegt nach 90minütiger Nachhydrierzeit bei 84% EDA sowie 6% DETA.

Aufgrund der FACH-Zugabe erfolgt eine die partielle Deaktivierung des Katalysators. AAN wird somit zu schnell zudosiert (AAN wird nach vollständiger Zugabe nachgewiesen), was sich in der Selektivität bemerkbar macht.

### Beispiel 6 (partielle Deaktivierung durch FACH)

In einem 270ml-Autoklaven werden 3,25g eines Cr-dotierten Raney-Kobalt-Katalysators sowie 15ml THF vorgelegt. Der Autoklav wird auf 100°C erwärmt und Wasserstoff bis zu einem Gesamtdruck von 50 bar aufgepresst. Innerhalb von 20 Minuten wird eine Mischung aus 13,8g (0,25mol) destilliertem AAN, 13,8g eines internen Standards sowie 4,2g Wasser, sowie 140mg an Formaldehydcyanhydrin in 106g THF zudosiert. Die Reaktionsmischung wird weitere 90 Minuten unter Reaktionsbedingungen gerührt. Zu unterschiedlichen Zeiträumen werden Proben genommen, die mittels Methanol homogenisiert werden. Nach vollständiger Zugabe kann kein AAN mehr nachgewiesen werden. Die Selektivität liegt nach 90minütiger Nachhydrierzeit bei 92% EDA sowie 2% DETA.

Durch Anpassung der Bedingungen (gegenüber Beispiel 5) wird schneller hydriert, und somit langsamer zudosiert als hydriert, weshalb die Selektivität erhöht ist.

### Vergleichsbeispiel 7 (partielle Deaktivierung durch FACH)

In einem 270ml-Autoklaven werden 3,25g eines Cr-dotierten Raney-Kobalt-Katalysators sowie 15ml THF vorgelegt. Der Autoklav wird auf 100°C erwärmt und Wasserstoff bis zu einem Gesamtdruck von 50 bar aufgepresst. Innerhalb von 20 Minuten wird eine Mischung aus 13,8g (0,25mol) destilliertem AAN, 13,8g eines internen Standards sowie 4,2g Wasser, sowie 1,4g an Formaldehydcyanhydrin in 106g THF zudosiert. Die Reaktionsmischung wird weitere 90 Minuten unter Reaktionsbedingungen gerührt. Zu unterschiedlichen Zeiträumen werden Proben genommen, die mittels Methanol homogenisiert werden. Nach vollständiger Zugabe kann noch 17% AAN nachgewiesen werden. Die Selektivität liegt nach 90minütiger Nachhydrierzeit bei 45% EDA sowie 15% DETA.

Hier wird gezeigt, dass infolge einer zu schnellen Zudosierung, größere Mengen an AAN übrig sind und nur geringe EDA-Konzentrationen erreicht werden.

### Vergleichsbeispiel 8 (partielle Deaktivierung durch FACH)

In einem 270ml-Autoklaven werden 3,25g eines Cr-dotierten Raney-Kobalt-Katalysators sowie 15ml THF vorgelegt. Der Autoklav wird auf 120°C erwärmt und Wasserstoff bis zu einem Gesamtdruck von 50 bar aufgepresst. Innerhalb von 20 Minuten wird eine Mischung aus 13,8g (0,25mol) destilliertem AAN, 13,8g eines internen Standards sowie 4,2g Wasser, sowie 1,4g an Formaldehydcyanhydrin in 106g THF zudosiert. Die Reaktionsmischung wird weitere 90 Minuten unter Reaktionsbedingungen gerührt. Zu unterschiedlichen Zeiträumen werden Proben genommen, die mittels Methanol homogenisiert werden. Nach vollständiger Zugabe kann noch 10% AAN nachgewiesen werden. Die Selektivität liegt nach 90minütiger Nachhydrierzeit bei 69% EDA sowie 8% DETA.

### Beispiel 9 (kontinuierliche Hydrierung 1 wasserfrei)

In einen 270ml-Autoklaven mit Stromstörern und Scheibenrührer werden 10 g Cr-dotierter Raney-Kobalt vorgelegt und kontinuierlich 50Nl (Normliter) Wasserstoff zugefahren. Eine 10,5 Gew.-%ige AAN-Lösung in THF wird kontinuierlich bei 170 bis 180 bar zugepumpt. Über eine Tauchfritte wird kontinuierlich Reaktionsgemisch ausgetragen. Die Reaktionstemperatur wird auf 120°C gehalten. Der Austrag wird über ein Regelventil entspannt. Die Zudosiermenge wird von 14g AAN bis auf 27g AAN pro Stunde erhöht. Regelmäßige Proben werden mittels GC analysiert. Zu keiner Zeit kann AAN im Austrag nachgewiesen werden. Bei der Zudosierung von 27gAAN/h werden Selektivitäten von 97 % EDA sowie 2 % DETA erhalten.

### Beispiel 10 (kontinuierliche Hydrierung / 60 Gew.% Wasser)

In einen 270ml-Autoklaven mit Stromstörern und Scheibenrührer werden 10g Cr-dotierter Raney-Kobalt vorgelegt und kontinuierlich 50Nl Wasserstoff zugefahren. Eine Mischung aus 40g AAN, 40g Diethylenglycoldimethylether (DEGDME) als interner Standard sowie 24g Wasser in 325g THF wird über 21 h kontinuierlich pro Stunde bei 50bar zugepumpt. Über eine Tauchfritte wird kontinuierlich Reaktionsgemisch ausgetragen. Die Reaktionstemperatur wird auf 120°C gehalten. Der Austrag wird über ein Regelventil entspannt. Regelmäßige Proben werden mittels GC analysiert. Zu keiner Zeit kann AAN im Austrag nachgewiesen werden. Die Proben zeigen gleich bleibend eine Selektivität von 96,5% EDA sowie 1,5% DETA.

### Beispiel 11 (kontinuierliche Hydrierung / 30 Gew.-% Wasser)

In einen 270ml-Autoklaven mit Stromstörern und Scheibenrührer werden10g Cr-dotierter Raney-Kobalt vorgelegt und kontinuierlich 50Nl Wasserstoff zugefahren. Eine Mischung aus 30g AAN, 9g Wasser in 255g THF wird kontinuierlich pro Stunde bei 50 bar zugepumpt. Über eine Tauchfritte wird kontinuierlich Reaktionsgemisch ausgetragen. Die Reaktionstemperatur wird auf 120°C gehalten. Der Austrag wird über ein Regelventil entspannt. Regelmäßige Proben werden mittels GC analysiert. Zu keiner Zeit kann AAN im Austrag nachgewiesen werden. Die Proben zeigen gleich bleibend eine Selektivität von >98 % EDA sowie 1% DETA.

## Patentansprüche

1. Verfahren zur Herstellung von Ethylendiamin durch Hydrierung von Aminoacetonitril an einem Katalysator, **dadurch gekennzeichnet, dass** die Hydrierung in einer Lösung durchgeführt wird, welche Aminoacetonitril, Wasser mit einem Anteil von 0 bis 60 Gew.-% und ein Lösungsmittel enthält, wobei das in der Lösung enthaltene Aminoacetonitril mit einer Rate in das Reaktionsgefäß zugeführt wird, die nicht größer als die Rate ist, mit der das Aminoacetonitril mit Wasserstoff bei der Hydrierung reagiert.

2. Verfahren zur Herstellung von Ethylendiamin nach Anspruch 1, **dadurch gekennzeichnet, dass** das Lösungsmittel ein Ether, mehr bevorzugt ein cyclischer Ether und besonders bevorzugt Tetrahydrofuran ist.

3. Verfahren zur Herstellung von Ethylendiamin nach Anspruch 1 oder 2, **dadurch gekennzeichnet, dass** das Aminoacetonitril des Gesamt-Frischzulaufs eine Konzentration von 5-30 Gew.-% aufweist.

4. Verfahren zur Herstellung von Ethylendiamin nach einem der Ansprüche 1 bis 3, **dadurch gekennzeichnet, dass** das Reaktionsgefäß ein Rührreaktor, ein Schlaufenreaktor oder ein sonstiger rückvermischter Reaktor ist, der in semikontinuierlicher oder in kontinuierlicher Fahrweise betrieben wird.

5. Verfahren zur Herstellung von Ethylendiamin nach einem der Ansprüche 1 bis 4, **dadurch gekennzeichnet, dass** der Katalysator ein Raney-Kobalt- oder Raney-NickelKatalysator und besonders bevorzugt ein mit Chrom dotierter Raney-Kobalt- oder Raney-Nickel-Katalysator ist.

6. Verfahren zur Herstellung von Ethylendiamin nach einem der Ansprüche 1 bis 5, **dadurch gekennzeichnet, dass** die Hydrierung bei einer Temperatur von 40 bis 150 °C, bevorzugt bei 75 bis 125 °C durchgeführt wird.

7. Verfahren zur Herstellung von Ethylendiamin nach einem der Ansprüche 1 bis 6, **dadurch gekennzeichnet, dass** die Hydrierung bei einem Druck von 5 bis 300 bar, bevorzugt von 30 bis 250 bar, besonders bevorzugt von 50 bis 160 bar durchgeführt wird.

8. Verfahren zur Herstellung von Ethylendiamin nach einem der Ansprüche 1 bis 7, **dadurch gekennzeichnet, dass** die Konzentration des Aminoacetonitrils im Reaktionsaustrag kleiner als 1 Gew.% ist.

9. Verfahren zur Herstellung von Ethylendiamin nach einem der Ansprüche 1 bis 8, **dadurch gekennzeichnet, dass** die Katalysatorbelastung bei 0,3 bis 20 g Aminonitril pro Gramm Katalysator pro Stunde liegt.

10. Verfahren zur Herstellung von Ethylendiamin nach einem der Ansprüche 1 bis 9, **dadurch gekennzeichnet, dass** die Hydrierung ohne Zusatz von Ammoniak durchgeführt wird.

11. Verfahren zur Herstellung von Ethylendiamin nach einem der Ansprüche 1 bis 10, **dadurch gekennzeichnet, dass** Aminoacetonitril eingesetzt wird, dass weitgehend frei von Formaldehydcyanhydrin (FACH) ist und/oder dass das Aminoacetonitril von der Hydrierung destilliert wurde.

## Claims

1. A process for preparing ethylenediamine by hydrogenation of aminoacetonitrile over a catalyst, wherein the hydrogenation is carried out in a solution comprising aminoacetonitrile, water in a proportion of from 0 to 60% by weight and a solvent and the aminoacetonitrile comprised in the solution is fed into the reaction vessel at a rate which is not greater than the rate at which the aminoacetonitrile reacts with hydrogen in the hydrogenation.

2. The process for preparing ethylenediamine according to claim 1, wherein the solvent is an ether, more preferably a cyclic ether and particularly preferably tetrahydrofuran.

3. The process for preparing ethylenediamine according to claim 1 or 2, wherein the aminoacetonitrile of the total fresh feed has a concentration of 5-30% by weight.

4. The process for preparing ethylenediamine according to any of claims 1 to 3, wherein the reaction vessel is a stirred reactor, a loop reactor or another backmixed reactor which is operated in semibatch or continuous mode.

5. The process for preparing ethylenediamine according to any of claims 1 to 4, wherein the catalyst is a Raney cobalt or Raney nickel catalyst and particularly preferably a chromium-doped Raney cobalt or Raney nickel catalyst.

6. The process for preparing ethylenediamine according to any of claims 1 to 5, wherein the hydrogenation is carried out at a temperature of from 40 to 150°C, preferably from 75 to 125°C.

7. The process for preparing ethylenediamine according to any of claims 1 to 6, wherein the hydrogenation is carried out at a pressure of from 5 to 300 bar, preferably from 30 to 250 bar, particularly preferably from 50 to 160 bar.

8. The process for preparing ethylenediamine according to any of claims 1 to 7, wherein the concentration of the aminoacetonitrile in the reaction product mixture is less than 1% by weight.

9. The process for preparing ethylenediamine according to any of claims 1 to 8, wherein the space velocity over the catalyst is from 0.3 to 20 g of amino nitrile per gram of catalyst per hour.

10. The process for preparing ethylenediamine according to any of claims 1 to 9, wherein the hydrogenation is carried out without addition of ammonia.

11. The process for preparing ethylenediamine according to any of claims 1 to 10, wherein aminoacetonitrile which is largely free of formaldehyde cyanohydrin (FACH) is used and/or the aminoacetonitrile has been distilled before the hydrogenation.

## Revendications

1. Procédé pour la préparation d'éthylènediamine par hydrogénation d'aminoacétonitrile sur un catalyseur, **caractérisé en ce que** l'hydrogénation est réalisée dans une solution qui contient de l'aminoacétonitrile, de l'eau à une proportion de 0 à 60 % en poids et un solvant, l'aminoacétonitrile contenu dans la solution étant introduit dans le récipient de réaction à une vitesse qui n'est pas supérieure à la vitesse à laquelle l'aminoacétonitrile réagit avec l'hydrogène lors de l'hydrogénation.

2. Procédé pour la préparation d'éthylènediamine selon la revendication 1, **caractérisé en ce que** le solvant est un éther, plus préférablement un éther cyclique et de manière particulièrement préférée le tétrahydrofuranne.

3. Procédé pour la préparation d'éthylènediamine selon la revendication 1 ou 2, **caractérisé en ce que** l'aminoacétonitrile de l'alimentation fraîche totale présente une concentration de 5-30% en poids.

4. Procédé pour la préparation d'éthylènediamine selon l'une quelconque des revendications 1 à 3, **caractérisé en ce que** le récipient de réaction est un réacteur agité, un réacteur à boucle à circulation interne ou un autre réacteur à mélange en retour qui est exploité en mode de fonctionnement semi-continu ou continu.

5. Procédé pour la préparation d'éthylènediamine selon l'une quelconque des revendications 1 à 4, **caractérisé en ce que** le catalyseur est un catalyseur de cobalt de Raney ou un catalyseur de nickel de Raney et de manière particulièrement préférée un catalyseur de cobalt de Raney ou de nickel de Raney dopé au chrome.

6. Procédé pour la préparation d'éthylènediamine selon l'une quelconque des revendications 1 à 5, **caractérisé en ce que** l'hydrogénation est réalisée à une température de 40°C à 150°C, de préférence de 75°C à 125°C.

7. Procédé pour la préparation d'éthylènediamine selon l'une quelconque des revendications 1 à 6, **caractérisé en ce que** l'hydrogénation est réalisée à une pression de 5 à 300 bars, de préférence de 30 à 250 bars, de manière particulièrement préférée de 50 à 160 bars.

8. Procédé pour la préparation d'éthylènediamine selon l'une quelconque des revendications 1 à 7, **caractérisé en ce que** la concentration en aminoacétonitrile dans le produit évacué de la réaction est inférieure à 1% en poids.

9. Procédé pour la préparation d'éthylènediamine selon l'une quelconque des revendications 1 à 8, **caractérisé en ce que** la charge du catalyseur est de 0,3 à 20 g d'aminonitrile par gramme de catalyseur par heure.

10. Procédé pour la préparation d'éthylènediamine selon l'une quelconque des revendications 1 à 9, **caractérisé en ce que** l'hydrogénation est réalisée sans addition d'ammoniac.

11. Procédé pour la préparation d'éthylènediamine selon l'une quelconque des revendications 1 à 10, **caractérisé en ce qu'**on utilise de l'aminoacétonitrile qui est dans une large mesure exempt de formaldéhydecyanhydrine (FACH) et/ou **en ce que** l'aminoacétonitrile a été distillé avant l'hydrogénation.
